# EUROPEAN PATENT APPLICATION

(11) **EP 1 470 830 A1**
(43) Date of publication of application: **27.10.2004**
(21) Application number: 04009726.3
(22) Date of filing: 23.04.2004
(51) Int. Cl.: A61L 31/10, A61L 31/16, A61L 29/08, A61L 27/34, A61F 2/06

(54) **Drug-polymer coated stent with polysulfone and styrenic block copolymer**

(30) Priority: 25.04.2003 US 465398 P
(71) Applicant: Medtronic Vascular, Inc., Santa Rosa, California 95403 (US)
(72) Inventor: Upidi, Kishore, Santa Rosa, CA 95403 (US); Cheng, Peiwen, Santa Rosa, CA 95409 (US); Patel, Kaushik A., Windsor, CA 95492 (US); Sundar, Rangarajan, Santa Rosa, CA 95404 (US)
(74) Representative: Hughes, Andrea Michelle

(57) **Abstract**

The present invention provides a system for treating a vascular condition, including a catheter 110; a stent 120 with a stent framework 122 that is coupled to the catheter; a polymeric coating 124 of a blended matrix of a polysulfone and a styrenic block copolymer that is disposed on the stent framework, and a therapeutic agent 126 in contact with the matrix. A drug-polymer coated stent, a method of manufacturing a drug-polymer coated stent, and method for treating a vascular condition are also disclosed.

## Description

### FIELD OF THE INVENTION

This invention relates generally to biomedical stents. More specifically, the invention relates to a drug-polymer coating comprising a blended matrix of a polysulfone and a styrenic block copolymer in contact with a therapeutic agent that are disposed on an endovascular stent for in vivo, timed-release drug delivery.

### BACKGROUND OF THE INVENTION

Stenting procedures have had major impact on the field of interventional cardiology and endovascular surgery. Yet, the success of stenting procedures is limited by in-stent restenosis and the increasing number of stent-induced lesions and neointimal formation that parallel the number of surgical procedures. The use of stents and stenting procedures has impacted many of the procedures in interventional cardiology and endovascular surgery. While stenting procedures have reduced the need for highly invasive surgery, problems of in-stent restenosis, stent-induced lesions and neointimal formation may occur as a result of these procedures. Much medical research and development in the last decade have been dedicated to endovascular stents, and in the most recent years, to drug-eluting coatings for stents. The efficacy of endovascular stents is potentially increased by the addition of stent coatings that include or encase pharmaceutical drugs and other therapeutic agents. These drugs may be released from the coatings while in the body, delivering their patent effects at the site where they are most needed. Thus, the localized levels of the medications can be elevated. The medications are therefore potentially more effective than orally- or intravenously-delivered drugs that distribute throughout the body, the latter which may have little effect on the impacted area or which may be expelled rapidly from the body without achieving their pharmaceutical intent. Furthermore, drugs released from tailored stent coatings may have controlled, timed-release qualities, eluting their bioactive agents over hours, weeks or even months.

Recent research has focused on stent coatings with various families of drug polymer chemistries that are used to increase the effectiveness of stenting procedures and control drug-elution properties. A polymeric coating of a polyamide, parylene or parylene derivative is disclosed by Ragheb et al. in "Coated Implantable Medical Device", US patent publication 2003/0036794, published February 20, 2003. Chudzik et al. presents a coating composition of a bioactive agent in combination with a mixture of a first polymer component such as poly(butyl methacrylate) and a second polymer component such as poly(ethylene-co-vinyl acetate) in "Bioactive Agent Release Coating", US patent publication 2003/0031780, published February 13, 2003. A composite polymer coating with a bioactive agent and a barrier coating formed in situ by a low energy plasma polymerization of a monomer gas is described in "Polymeric Coatings for Controlled Delivery of Active Agents," K. R. Kamath, US patent number 6,335,029 issued January 1, 2002. A polymeric coating for an implantable medical article based on hydrophobic methacrylate and acrylate monomers, a functional monomer having pendant chemically reactive amino groups capable of forming covalent bonds with biologically active compounds, and a hydrophilic monomer wherein a biomolecule is coupled to the coated surface, is presented in "Implantable Medical Device," E. Koulik, et al., US patent number 6,270,788, issued Aug. 7, 2001. Yang et al. discloses a polymeric coated stent with a first blended polymeric material of a faster releasing PLA-PEO copolymer and a slower releasing PLA-PCL copolymer in "Stent Coating", US patent number 6,258,121, issued July 10, 2001. Use of block copolymers on a hydrophobic polymer substrate is described in "Biocompatible Polymer Articles," E. Ruckenstein, et al., US patent number 4,929,510, issued May 29, 1990. A method for the volumetric inclusion and grafting of hydrophilic compounds in a hydrophobic substrate using an irradiation means is described in "Hydrophobic Substrate with Grafted Hydrophilic Inclusions," G. Gaussens, et al., US patent number 4,196,065, issued Apr. 1, 1980.

When selecting polymers for drug delivery, it is important to consider their biocompatibility and biostability, their satisfactory mechanical properties such as durability and integrity during roll down and expansion of the stent, and their release profiles for the drugs. Polymer biocompatibility can be determined by in-vitro studies such as cytotoxicity and hemolysis, and in-vivo studies such as rabbit iliac implantation, 30-day swine implantation and 90-day mini-swine implantation.

Candidate chemistries for drug polymers may result in excessively rapid elution of an incorporated drug. When a drug is eluted too quickly, it may be ineffective and exceed dosage limits. If a drug is eluted too slowly, the pharmaceutical intent may remain unfulfilled. Furthermore, incorporation of more than one drug in the same coating can result in a much faster elution rate of a second drug in the same drug polymer, making the controlled delivery of multiple drugs difficult. Even pharmaceutical compounds with essentially the same pharmaceutical effect can have dramatically different elution rates in the same coating chemistry, depending on the formation of the compounds. Drug elution rates may be monitored with ultraviolet-visible spectroscopy (UV-VIS) and high-performance liquid chromatography (HPLC).

Drug polymers and coatings need to have intrinsic mechanical flexibility if they are to be used effectively on a stent. A stent may be deployed by self-expansion or balloon expansion. Either deployment method may be accompanied by a high level of bending at portions of the stent framework, which can cause cracking, flaking, peeling, or delaminating of many candidate drug polymers. Deployment may increase the stent diameter by threefold or more during expansion. The candidate drug polymer may not stick or adhere. Furthermore, the coating may fall off, crystallize or melt during preparation and sterilization prior to deployment, further limiting the types of drug polymers and polymer coatings acceptable for use on cardiovascular stents. Stent roll down, expansion, and simulated lesion abrasion testing are often used to test mechanical properties. The coating integrity is observed by optical and scanning electron microscopy. A beneficial drug-polymer system is one that can be tailored to provide a desired elution rate for a specific drug. Improved stenting procedures would employ a drug-polymer system that can be tailored to accommodate a variety of drugs for controlled time delivery, while maintaining mechanical integrity during stent preparation and deployment. A polymeric system that can be readily altered to control the elution rate of interdispersed bioactive drugs and to control their bioavailability provides further benefit.

Therefore, a desirable drug-polymer system provides a convenient, flexible and biocompatible polymer chemistry for drug-polymer coated stents and other implanted articles, while overcoming the deficiencies and limitations described above.

### SUMMARY OF THE INVENTION

One aspect of the invention provides a system for treating a vascular condition, including a catheter; a stent with a stent framework that is coupled to the catheter; a polymeric coating of a blended matrix of a polysulfone and a styrenic block copolymer that is disposed on the stent framework. A therapeutic agent is in contact with the blended matrix.

Another aspect of the invention is a method of manufacturing a drug-polymer coated stent, including the steps of forming a polymeric solution of a styrenic block copolymer and a styrenic block copolymer solvent; adding a polysulfone to the polymeric solution to form a blended matrix of the polysulfone and the styrenic block copolymer; applying the polymeric solution onto a stent framework; and drying the polymeric solution.

Another aspect of the invention provides a drug-polymer coated stent, comprising a stent framework and a polymeric coating disposed on the stent framework, and a therapeutic agent contacting the polymeric coating. The polymeric coating comprises a blended matrix of a polysulfone and a styrenic block copolymer.

The present invention is illustrated by the accompanying drawings of various embodiments and the detailed description given below. The drawings should not be taken to limit the invention to the specific embodiments, but are for explanation and understanding. The detailed description and drawings are merely illustrative of the invention rather than limiting, the scope of the invention being defined by the appended claims. The foregoing aspects and other attendant advantages of the present invention will become more readily appreciated by the detailed description taken in conjunction with the accompanying drawings.

### BRIEF DESCRIPTION OF THE DRAWINGS

Various embodiment of the present invention are illustrated by the accompanying figures, wherein:
FIG. 1 is an illustration of a system for treating a vascular condition, in accordance with one embodiment of the current invention;
FIG. 2 is a cross-sectional view a drug-polymer coated stent, in accordance with one embodiment of the current invention;
FIG. 3 is a schematic illustration of a blended matrix of a polysulfone and a styrenic block copolymer; in accordance with one embodiment of the current invention;
FIG. 4a is a graph of a drug elution rate from a drug-polymer coated stent, in accordance with one embodiment of the current invention;
FIG. 4b is a graph of drug elution from a drug-polymer coated stent, in accordance with one embodiment of the current invention;
FIG. 5 is a graphical illustration of drug elution from a drug-polymer coated stent with a tailored blend of a polysulfone and a styrenic block copolymer, in accordance with one embodiment of the current invention;
FIG. 6 is a flow diagram of a method of manufacturing a drug-polymer coated stent, in accordance with one embodiment of the current invention; and
FIG. 7 is a flow diagram of a method of treating a vascular condition, in accordance with one embodiment of the current invention.

### DETAILED DESCRIPTION OF THE PRESENTLY PREFERRED EMBODIMENTS

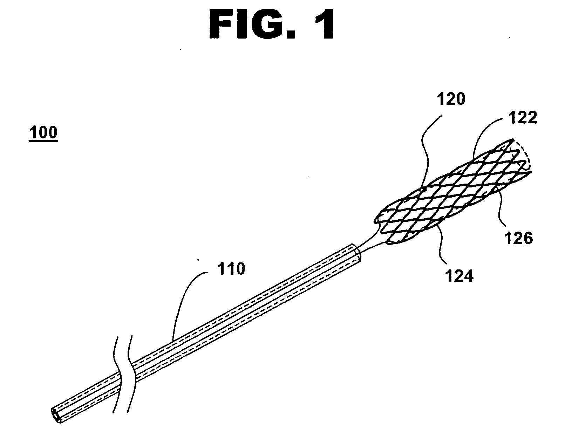

FIG. 1 shows an illustration of a system for treating a vascular condition, in accordance with one embodiment of the present invention at 100. Vascular condition treatment system 100 includes a catheter 110, a stent 120 with a stent framework 122 coupled to the catheter, and a polymeric coating 124 disposed on stent framework 122. A therapeutic agent 126 is in contact with polymeric coating 124. Vascular condition treatment system 100 may be used, for example, to treat heart disease, various cardiovascular ailments, and other vascular conditions using catheter-deployed endovascular stents with tailored polymeric coatings for controlling the timed-release properties of interdispersed or encased therapeutic agents. Treatment of vascular conditions may include the prevention or correction of various ailments and deficiencies associated with the cardiovascular system, urinogenital systems, biliary conduits, abdominal passageways and other biological vessels within the body.

Polymeric coating 124 comprises a blended matrix of a polysulfone polymer and a styrenic block copolymer. One or more therapeutic agents 126 may be dispersed throughout polymeric coating 124. Alternatively, therapeutic agents 126 may be positioned between polymeric coating 124 and stent framework 122. Therapeutic agent 126 may be a pharmacologically active drug or bioactive compound. The blended matrix controls the elution rate of therapeutic agent 126, and provides a controlled drug-elution characteristic. Drug elution refers to the transfer of a therapeutic agent from polymeric coating 124. The elution is determined as the total amount of therapeutic agent excreted out of polymeric coating 124, typically measured in units of weight such as micrograms, or in weight per peripheral area of the stent. In one embodiment, polymeric coating 124 includes between 0.1 percent and 50 percent of therapeutic agent 126 by weight. In another embodiment, polymeric coating 124 has less than 0.1 percent or zero therapeutic compounds dispersed within polymeric coating 124, though therapeutic agent 126 is in contact with and encased by polymeric coating 124, positioned between stent framework 122 and polymeric coating 124. Polymeric coating 124 serves as a cap coating or a barrier coating. The cap coat may have a thickness, for example, between one and 5 microns or larger.

Blended polysulfone polymers and styrenic block copolymers such as Kraton-G® or Kraton-D® provide coatings with good overall property balance, particularly with respect to durability and drug elution. Polysulfone resins are high molecular weight, linear heterochain polymers. They are amorphous, have a relatively high glass transition temperature (Tg), and provide tailorable mechanical and barrier properties. Polysulfone resins are also tough and rigid, and in high fractions, they result in increased hardness and lower elution rates.

Styrenic block copolymers such as Kraton® resins are thermoplastic elastomers with styrene end blocks and saturated or unsaturated mid-blocks. In terms of molecular structure, Kraton® resins are anionically polymerized block copolymers with hydrophobic and hydrophilic phase separation properties. Styrenic block copolymers such as Kraton-G® or Kraton-D® provide coatings with good overall property balance, particularly with respect to durability and drug elution. Kraton® polymers such as Kraton-G® and Kraton-D® are manufactured by Kraton Polymers® of Houston, TX. Kraton-G® polymers have a saturated mid-block such as an ethylene and butylene random copolymer (SEBS) or an ethylene and propylene chain (SEPS). Kraton-D® polymers have an unsaturated rubbery mid-block such as butadiene (SBS) or isoprene (SIS). The rubbery mid-blocks offer good impact resistance properties at low and ambient temperatures. The relatively low glass transition temperature (Tg) also gives better adhesive properties and relatively fast drug release. In addition, Kraton® resins have very good compatibility with a wide range of polymers to allow combinations of properties that could not be achieved otherwise.

By tailoring polysulfone and Kraton blends, desired drug release properties and good mechanical properties can be achieved in a coating for blood-contacting biomedical implants such as stents. Metal-adhering attributes such as hydrophilicity aid in the cohesiveness of the polymers to metallic stents, whereas hydrophobic attributes assist in the timed-release control of pharmaceutical compounds interdispersed within or encased by the drug-polymer coating. By tailoring the fractional constituency of the polysulfone and the styrenic block copolymer, the concentration, distribution profile, and elution rates of therapeutic agents can be controlled. The elution rates of the therapeutic agents are affected by the chain length of the styrenic block copolymer, the chain length of the polysulfone, and the structure of the therapeutic agents, among others. The formulations and fractional composition of the blended polymers may be selected to provide desired elution rates of embedded or encased therapeutic agents.

Upon insertion of catheter 110 and stent 120 with polymeric coating 124 into a directed vascular region of a human body, stent 120 may be expanded by applying pressure to a suitable balloon inside stent 120, or by retracting a sheath to allow expansion of a self-expanding stent 120. Balloon deployment of stents and self-expanding stents are well known in the art. Catheter 110 may include the balloon used to expand stent 120. Catheter 110 may include a sheath that retracts to allow expansion of a self-expanding stent.

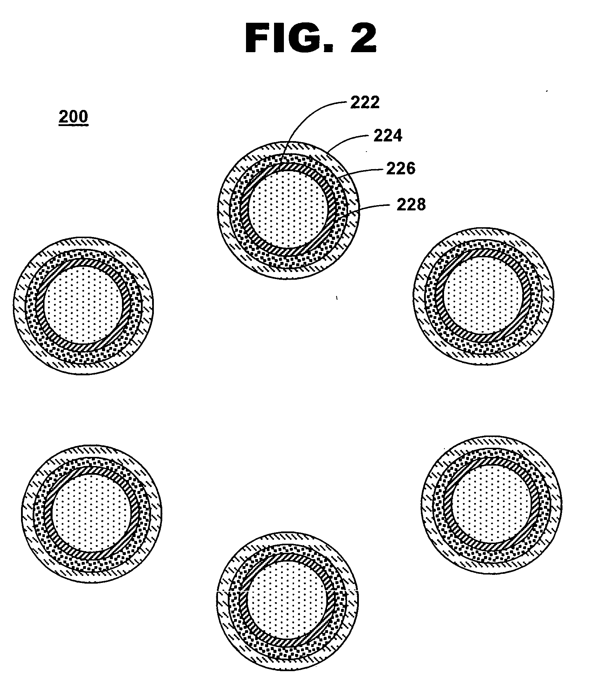

FIG. 2 shows a cross-sectional view a drug-polymer coated stent, in accordance with one embodiment of the present invention at 200. Drug-polymer coated stent 200 includes a polymeric coating 224 disposed on a stent framework 222. Polymeric coating 224 includes a polymeric blend of a polysulfone polymer and a styrenic block copolymer forming a blended matrix, with one or more therapeutic agents 226 in contact with the blended matrix. Therapeutic agents 226 may be dispersed within the blended matrix of the polysulfone and the styrenic block copolymer, contained in one or more layers positioned between polymeric coating 224 and stent framework 222, or a combination thereof. Tailoring the fraction of the polysulfone polymer and the styrenic block copolymer in polymeric coating 224 controls the elution rate of one or more therapeutic agents dispersed within or encased by polymeric coating 224.

Stent framework 222 typically includes a metallic or a polymeric base. The metallic base comprises a metal such as stainless steel, nitinol, tantalum, MP35N alloy, platinum, titanium, a suitable biocompatible alloy, a suitable biocompatible material, or a combination thereof. The polymeric base material may comprise any suitable polymer for biomedical stent applications, as is known in the art.

Drug-polymer coated stent 200 includes one or more polymeric coatings 224 on stent framework 222. A primer coating 228, also referred to as an adhesive coating or a barrier coating, may be positioned between stent framework 222 and polymeric coating 224.

Polymeric coating 224 may have a predominantly hydrophilic characteristic to improve metal adhesion and, in some cases, to enhance the elution of embedded therapeutic material. Polymeric coating 224 may also have a hydrophobic characteristic. A relatively hydrophobic characteristic usually slows or mitigates the elution of the therapeutic agents and polymeric material into the body, and provides a tailored barrier for the elution of therapeutic material from or through the polymeric coating. The blended matrix of polysulfone and styrenic block copolymer in polymeric coating 224 provides a controlled elution rate for the therapeutic agent.

Polymeric coating 224 may include or encapsulate one or more therapeutic agents 226. The therapeutic agent is an agent capable of producing a beneficial affect against one or more conditions including coronary restenosis, cardiovascular restenosis, angiographic restenosis, arteriosclerosis, hyperplasia, and other diseases and conditions. For example, the therapeutic agent can be selected to inhibit or prevent vascular restenosis, a condition corresponding to a narrowing or constriction of the diameter of the bodily lumen where the stent is placed. In one embodiment, the therapeutic agent comprises an antirestenotic drug. In other embodiments, the therapeutic agent comprises an antisense agent, an antineoplastic agent, an antiproliferative agent, an antithrombogenic agent, an anticoagulant, an antiplatelet agent, an antibiotic, an anti-inflammatory agent, a steroid, a gene therapy agent, a therapeutic substance, an organic drug, a pharmaceutical compound, a recombinant DNA product, a recombinant RNA product, a collagen, a collagenic derivative, a protein, a protein analog, a saccharide, a saccharide derivative, and combinations thereof. In another embodiment, polymeric coating 224 includes a combination or cocktail of therapeutic agents. Therapeutic agent 226 may comprise, for example, a bioactive agent or a pharmaceutical drug.

A number of pharmaceutical drugs have the potential to be used in drug-polymer coatings. For example, an antirestenotic agent such as rapamycin or a rapamycin analog prevents or reduces the recurrence of narrowing and blockage of the bodily vessel. An antisense drug works at the genetic level to interrupt the process by which disease-causing proteins are produced. An antineoplastic agent is typically used to prevent, kill, or block the growth and spread of cancer cells in the vicinity of the stent. An antiproliferative agent may prevent or stop targeted cells or cell types from growing. An antithrombogenic agent actively retards blood clot formation. An anticoagulant often delays or prevents blood coagulation with anticoagulant therapy by using compounds such as heparin and coumarins. An antiplatelet agent may be used to act upon blood platelets, inhibiting their function in blood coagulation. An antibiotic is frequently employed to kill or inhibit the growth of microorganisms and to combat disease and infection. An anti-inflammatory agent such as dexamethasone can be used to counteract or reduce inflammation in the vicinity of the stent. In select cases, a steroid is used to reduce scar tissue in proximity to an implanted stent. A gene therapy agent may be capable of changing the expression of a person's genes to treat, cure or ultimately prevent disease. An organic drug is any small-molecule therapeutic material. A pharmaceutical compound is any compound that provides a therapeutic effect. A recombinant DNA product or a recombinant RNA product includes altered DNA or RNA genetic material. Therapeutic agents of pharmaceutical value may also include collagen and other proteins, saccharides, and their derivatives.

Polymeric coating 224 elutes at least one therapeutic agent 226. Polymeric coating 224 may include and elute multiple therapeutic agents 226. The relative concentrations of polysulfone and the styrenic block copolymer can be tailored to control the elution of one or more therapeutic agents 226 from drug-polymer coated stent 200. Elution of therapeutic agents 226 occurs primarily by diffusion processes. In some cases, a portion of polymeric coating 224 is absorbed into the body to release therapeutic agents 226 from within the coating. In other cases, a portion of polymeric coating 224 erodes away to release therapeutic agents 226.

Polymeric coating 224 contains a blended matrix wherein the blended matrix comprises fractional parts of polysulfone and styrenic block copolymer based on a predetermined elution rate of the therapeutic agent. Modification of the blended matrix allows, for example, rapid delivery of a pharmacologically active drug or bioactive agent within twenty-four hours of surgery, with a slower, steady delivery of a second bioactive agent over the next three to six months.

Polymeric coating 224 may include a plurality of therapeutic agents 226 with each therapeutic agent 226 having a predetermined elution rate. The blended matrix of the polysulfone and the styrenic block copolymer elutes the therapeutic agents at the predetermined elution rates. In one example, polymeric coating 224 includes a first therapeutic agent 226 concentrated adjacent to stent framework 222, and a second therapeutic agent 226 concentrated adjacent to the outer surface of polymeric coating 224. The first therapeutic agent 226 may comprise, for example, an antirestenotic drug and the second therapeutic agent 226 may comprise, for example, an anti-inflammatory drug.

In cases where the blended matrix of polysulfone and the styrenic block copolymer provides inadequate adhesion to the underlying metallic stent framework, an intermediate adhesion layer or primer coating 228 may be incorporated between the drug-polymer coating and the stent framework.

Drug-polymer coated stent 200 includes one or more polymeric coatings 224 on stent framework 222. Adhesive coating or primer coating 228 may be disposed on stent framework 222 and positioned between stent framework 222 and polymeric coating 224 to improve the adhesion of polymeric coating 224 and its durability. Primer coating 228 may be a polymeric material or any material that adheres well to the underlying stent, particularly when the stent has a metallic framework. Primer coating 228 is selected to adhere well to the stent and to be readily coated with another polymeric material such as polymeric coating 224 or an intervening layer comprising therapeutic agent 226. Primer coating 228 may be any suitable polymeric primer material such as parylene, polyurethane, phenoxy, epoxy, polyimide, polysulfone, or pellathane.

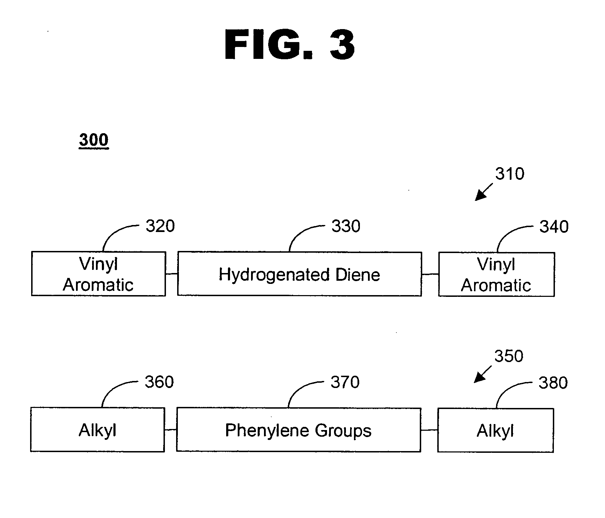

FIG. 3 shows a schematic illustration of a blended matrix of a polysulfone and a styrenic block copolymer, in accordance with the present invention at 300. Blended matrix 300 includes a styrenic block copolymer 310 and a polysulfone 350. Styrenic block copolymer 310 contains two end-blocks 320 and 340 on each end of a mid-block 330. End-blocks 320 and 340 are vinyl aromatics such as styrene, and mid-block 330 comprising a hydrogenated diene. The hydrogenated diene can be saturated or unsaturated. Styrenic block copolymer 310 may comprise, for example, styrene-ethylene/butylene-styrene, where the ethylene/butylene is a random copolymer linear chain. An example of a styrenic block copolymer with a saturated mid-block is styrene-ethylene/propylene-styrene, where the ethylene/propylene represents a linear copolymer chain between two end-blocks of styrene. Styrenic block copolymer 310 may comprise vinyl aromatic end-blocks 320 and 340 with an unsaturated diene, such as styrene-butadiene-styrene or styrene-isoprene-styrene. The styrenic block copolymer has a molecular weight between 200 Daltons and 200,000 Daltons, depending on the length of the block copolymer and the desired elution characteristics.

Polysulfone 350 is a heterochain polymer with a plurality of phenylene groups 370, typically terminated with alkyl end-groups 360 and 380. The polysulfone polymer generally has a molecular weight between 10,000 Daltons and 100,000 Daltons.

The styrenic block copolymer is combined with the polysulfone polymer to form a blended matrix. The blended matrix provides a controlled drug-elution characteristic, with a higher fraction of polysulfone polymer typically corresponding to a lower elution rate, and a higher fraction of styrenic block copolymer corresponding to a higher elution rate of an interdispersed or encased therapeutic agent. The blended matrix may comprise, for example, between 10 percent and 90 percent polysulfone polymer by volume. The blended matrix may comprise, for example, between 10 percent and 90 percent styrenic block copolymer by volume.

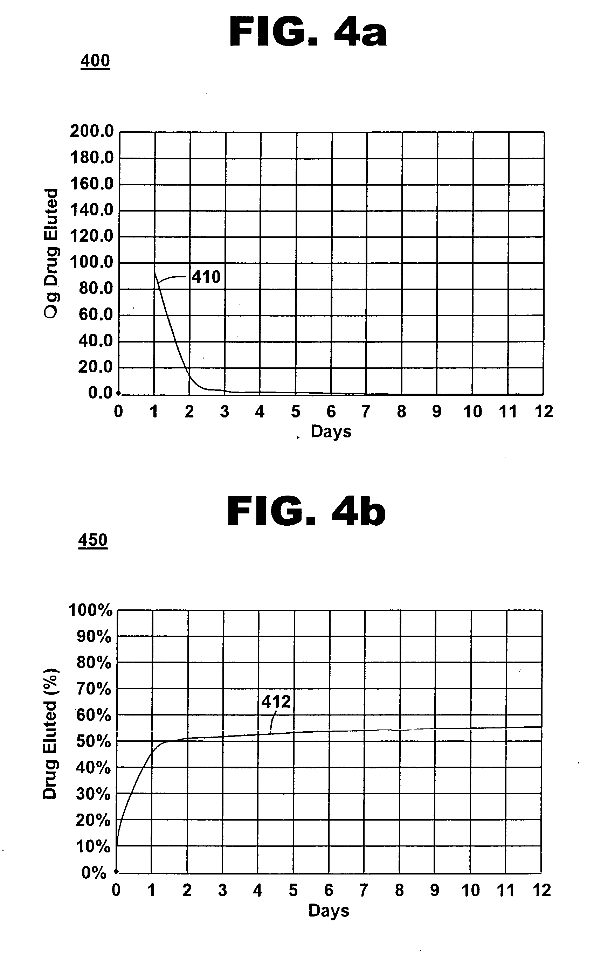

FIG. 4a shows a graph of a drug elution rate from a drug-polymer coated stent, in accordance with one embodiment of the present invention at 400. Elution graph 400 shows the rate of an exemplary antirestenotic drug eluted from a drug-polymer coated stent as a function of time. The antirestenotic drug, a rapamycin analog, comprises 25% of the polymer coating by weight on an 18 millimeter expanded stent. The weight of the base coat is initially 787 micrograms, and no cap coating is used. Drug elution rates are monitored with high-performance liquid chromatography (HPLC). The elution of the therapeutic agent is indicated as the weight of drug eluted from the stent coating, measured in micrograms of drug eluted per day. The elution rate profile 410 of the drug shows a high rate of drug delivery over an initial period of two days or so after stent deployment, with minimal drug eluted over the next several weeks. The elution rate is determined from a typical elution graph 450 by taking the derivative with respect to time, or by dividing the total amount of drug eluted by the elapsed time since stent deployment.

FIG. 4b shows a graph of drug elution from a drug-polymer coated stent, in accordance with one embodiment of the present invention at 450. Elution graph 450 shows the elution of an antirestenotic drug from a drug?polymer coated stent as a function of time. The elution of the therapeutic agent is indicated as a percentage by weight of total drug initially dispersed within the stent coating. Typical units used for drug elution include micrograms of drug. Alternatively, they can be normalized to a unit volume with units such as micrograms per cubic centimeter of drug-polymer, or normalized to the periphery area of the stent with units such as micrograms per square centimeter. The elution profile 412 of the drug shows a high rate of drug delivery over an initial period of two days or so after stent deployment, with minimal drug eluted over the following several weeks. Selection of the appropriate drug, the fractional portion of the polysulfone polymer and the styrenic block copolymer in the blended matrix, as well as the method of preparation establish the elution profile of the therapeutic agent.

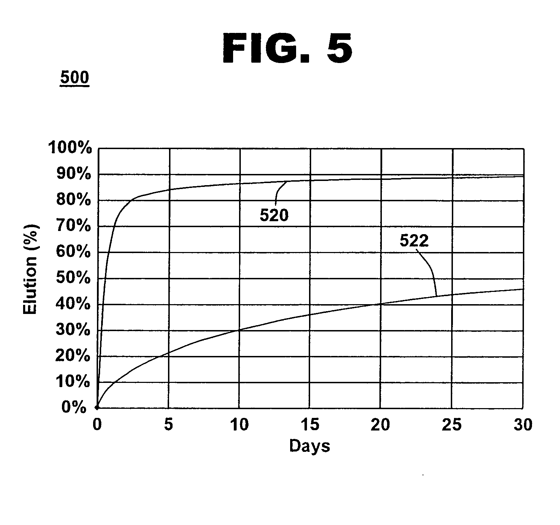

FIG. 5 shows a graphical illustration of drug elution from a drug-polymer coated stent with a tailored blend of a polysulfone and a styrenic block copolymer, in accordance with one embodiment of the present invention at 500. Elution graph 500 shows the elution of two therapeutic agents from a drug-polymer coated stent as a function of time. The elution of the therapeutic agents is indicated as a percentage with respect to the weight of each therapeutic agent dispersed within the stent coating or encased by the polymeric coating. Elution profile 520 of the first drug shows a high rate of drug delivery over an initial period of two days or so after stent deployment, with minimal drug eluted over the remainder of the month. Elution profile 522 of the second drug shows a slow initial rate of drug delivery, and steady delivery of the drug over an extended period of time. The elution profile of the therapeutic agents can be tailored to establish and control the elution rate through the selection of appropriate drugs and other therapeutic agents, the chain length of the polysulfone, the chain length of the styrenic block copolymer, the fraction of polysulfone and styrenic block copolymer, the distribution profile of the therapeutic agent within the polymeric coating, the concentration and amount of drug encompassed by the polymeric coating, among other variables.

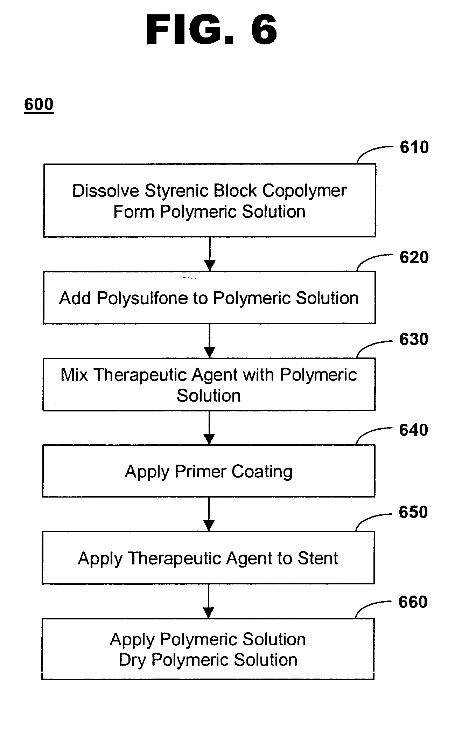

FIG. 6 shows a flow diagram of a method of manufacturing a drug-polymer stent including a therapeutic agent and a blended polymer of polysulfone and a styrenic block copolymer, in accordance with the present invention at 600. Drug-coated stent manufacturing method 600 comprises steps to form a drug-polymer coated stent containing polysulfone polymers, styrenic block copolymers, and one or more pharmaceutical agents in contact with the blended matrix of polysulfone and styrenic block copolymer.

In this embodiment, a styrenic block copolymer resin or a styrenic block copolymer is dissolved in a styrenic block copolymer solvent to form a polymeric solution, as seen at block 610. A styrenic block copolymer such as Kraton-G® or Kraton-D® or a derivative thereof is dissolved in a styrenic block copolymer solvent to form a polymeric solution. The styrenic block copolymer has a molecular weight, for example, between 200 Daltons and 200,000 Daltons. The content of the styrenic block copolymer in the polymeric solution is determined by the desired solids content, which may be less than one percent or as high as five percent by volume. The styrenic block copolymer solvent is any suitable organic solvent capable of dissolving the styrenic block copolymer such as chloroform, methyl ethyl ketone, tetrahydrofuran, methyl chloride, toluene, ethyl acetate or dioxane.

The polysulfone is added and mixed with the polymeric solution to form a polymeric solution with a blended matrix of polysulfone and styrenic block copolymer, as seen at block 620. The styrenic block copolymer may be added directly to the polymeric solution and mixed. Alternatively, the polysulfone may be dissolved and premixed in a suitable organic solvent such as chloroform, methyl ethyl ketone, tetrahydrofuran, methyl chloride, toluene, ethyl acetate or dioxane, and then added to the polymeric solution. The polysulfone has a molecular weight, for example, between 10,000 Daltons and 100,000 Daltons. The polysulfone content is usually less than one percent or as high as five percent by volume. The polysulfone typically comprises between 10 percent and 90 percent of the solids content in the polymeric solution by volume. The polysulfone polymer may comprise between 10 percent and 90 percent of the solids content in the polymeric solution by volume. The fractional percentage of the polysulfone and the styrenic block copolymer provides a controlled drug-elution characteristic, and may be selected to provide a predetermined elution rate.

One or more therapeutic agents may be mixed with the polymeric solution prior to applying the polymeric solution onto the stent framework, as seen at block 630. The therapeutic agents may be added directly into the polymeric solution and mixed. Alternatively, the therapeutic agents may be dissolved in a therapeutic agent solution comprising a suitable solvent, then added and mixed with the polymeric solution. The therapeutic agent constituency of the polymeric coating is usually between 0.1 percent and 50 percent of the therapeutic agent by weight.

A primer coating may be applied to the metallic or polymeric stent framework, as seen at block 640. The primer coating may be applied onto the stent framework prior to applying the polymeric solution onto the stent framework so as to improve adhesion, particularly to metal stents such as stainless steel. The primer coating comprises any suitable primer material such as parylene, polyurethane, phenoxy, epoxy, polyimide, polysulfone, or pellathane. The primer coating may be applied to the stent framework by dipping, spraying, painting, brushing, or other suitable methods. Prior to primer coating application, the stent may be cleaned using, for example, various degreasers, solvents, surfactants and de-ionized water, as is known in the art.

The primer coating is dried and cured or cross-linked as needed. Excess liquid may be blown off prior to drying the primer coating. Drying of the primer coating to eliminate or remove any volatile components may be done at room temperature or elevated temperatures under a dry nitrogen or other suitable environments including a vacuum environment. During the coating process, the primer coating and any curing agents may react at room temperature. After coating, the coated stents may be raised to an elevated temperature to increase the reaction rates between the primer and any curing agents. The primer-coated stent may be baked at elevated temperatures on the order of 150 to 200 degrees centigrade to drive off any solvent trapped inside the primer coating and to cure the primer coating by providing thermal energy for cross linking the primer coating with the cross-linking agent. Full curing of the primer coating is desired so that solvents used for therapeutic agent application and for polymeric coating application do not significantly degrade the primer coating, and so that high-temperature processing is not needed for drug-polymer applications, which may degrade the drugs.

A second dipping and drying step may be used to thicken the coating when needed. The thickness of the primer coating may range between 0.1 microns (micrometers) and 2.0 microns or greater in order to adequately coat and protect the stent framework, and to provide a satisfactory underlayer for subsequent therapeutic agent and polymeric coating applications. The weight of the primer coating depends on the diameter and length of the stent, though a typical weight of the primer coating is between 20 micrograms and 200 micrograms. Additional application and drying steps may be included to reach the desired thickness of the primer coating and to ensure adequate coverage of the stent framework.

One or more therapeutic agents may be applied to the stent framework prior to applying the polymeric solution onto the stent framework, as seen at block 650. The therapeutic agents may be applied using any suitable application technique such as dipping, spraying, painting, or brushing. The therapeutic agents are dried after application by evaporating off any solvents. The layer of therapeutic agents may be applied with or without polysulfone and styrenic block copolymers. The thickness of the therapeutic agent positioned between the polymeric coating and the stent framework may range between 0.1 microns and 20 microns or greater in order to provide the desired therapeutic effect.

The polymeric solution is then applied to the stent framework and dried, as seen at block 660. The polymeric solution is applied by using an application technique such as dipping, spraying, painting or brushing. The polymeric solution is generally dried after application by evaporating off the solvent at room temperature and under ambient conditions. A nitrogen environment or other controlled environment may also be used. Alternatively, the drug-polymer solution can be dried by evaporating the majority of the solvent at room temperature, and then further dried in a vacuum environment between, for example, room temperature of about 25 degrees centigrade and 45 degrees centigrade or higher to extract any pockets of solvent buried within the polymeric coating.

The thickness of the polymeric coating can vary, though is typically between 0.5 micron and 20 microns. Depending on the diameter and length of the stent, the weight of the polymeric coating is usually between 50 micrograms and 1500 micrograms for a range of stent sizes. Additional polymeric coats may be added to thicken the drug coating. The additional polymeric coatings may have different concentrations or types of therapeutic agents. One or more barrier coatings may be positioned between composite polymeric coatings to aid in the control of the elution rate of one or more therapeutic agents dispersed within or encased by the polymeric coatings.

Variants of the method for manufacturing a drug-polymer coated stent can be employed, such as initially mixing the polysulfone, styrenic block copolymer and any therapeutic agents into the same solvent, using separate solvents for each component, or altering the order of mixing the stock solutions.

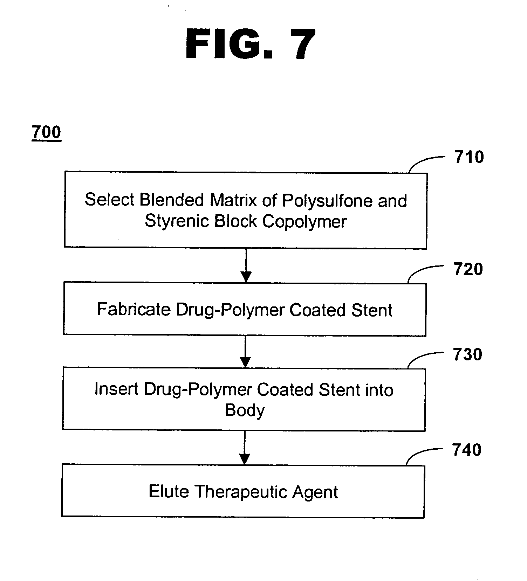

FIG. 7 shows a flow diagram of a method for treating a vascular condition, using an embodiment of the present invention at 700. Vascular condition treatment method 700 includes steps to insert a drug-polymer coated stent within a vessel of a body and to elute at least one therapeutic agent from the drug-polymer coated stent into the body. One or more therapeutic agents are in contact with a blended matrix of a polysulfone and a styrenic block copolymer.

The fractional constituencies of the blended matrix of polysulfone and styrenic block copolymer are selected to achieve an intended pharmaceutical intent, such as a predetermined elution rate for one or more therapeutic agents in contact with the polymeric coating, as seen at block 710. One or more therapeutic agents may be added to the blended matrix. Alternatively, one or more therapeutic agents may be applied to the stent framework, and then encased with the blended matrix of polysulfone and styrenic block copolymer. The blended matrix of the polysulfone and the styrenic block copolymer may control the elution rate of each therapeutic agent.

A drug-polymer coated stent is fabricated with the selected blended matrix and therapeutic agents, as seen at block 720. The stent is coated with the polymeric coating, and dried. In one example, the polymeric coating includes one or more therapeutic agents dispersed within a blended matrix of a polysulfone polymer and a styrenic block copolymer. In another example, the polymeric coating comprises a polysulfone polymer and a styrenic block copolymer without any therapeutic agents, and encases a layer of one or more therapeutic agents disposed on the stent framework. A primer coating may be included to improve the adhesion between the stent framework and the coatings.

In one exemplary method, finished coated stents are reduced in diameter and placed into the distal end of the catheter in a process that forms an interference fit, which secures the stent onto the catheter. The catheter with the stent may be placed in a catheter package and sterilized prior to shipping and storing. Sterilization of the stent using conventional means is completed before clinical use.

When ready for deployment, the drug-polymer coated stent including a therapeutic agent and the selected blended matrix is inserted into a vessel of the body, as seen at block 730. The drug-coated stent is inserted typically in a controlled environment such as a catheter lab or hospital. The stent is deployed, for example, by expanding the stent with a balloon or by extracting a sheath to allow a self-expandable stent to enlarge after positioning the stent at a desired location within the body.

Once deployed, the therapeutic compounds in the polymeric coating or encased by the polymeric coating are eluted into the body, as seen at block 740. The elution rates of the therapeutic agents into the body and the tissue bed surrounding the stent framework are based on the fractional constituency of the blended matrix and the selected therapeutic agents, among other factors.

Although the present invention applies to cardiovascular and endovascular stents with timed-release therapeutic agents, the use of polymeric blends of polysulfone polymer and styrenic block copolymers with therapeutic agents dispersed within the polymeric coating or encased therein by the polymeric coating may be applied to other implantable and blood-contacting biomedical devices such as coated pacemaker leads, microdelivery pumps, feeding and delivery catheters, heart valves, artificial livers and other artificial organs.

## Claims

1. A system for treating a vascular condition, comprising:
a catheter (110);
a stent (120) coupled to the catheter, the stent including a stent framework (122);
a polymeric coating (124) disposed on the stent framework, wherein the polymeric coating comprises a blended matrix of a polysulfone and a styrenic block copolymer; and
a therapeutic agent (126) in contact with the blended matrix.

2. The system of claim 1 wherein the catheter (110) includes a balloon used to expand the stent (120).

3. The system of claim 1 wherein the catheter (110) includes a sheath that retracts to allow expansion of the stent (120).

4. The system of claim 1 wherein the stent framework (122) comprises one of a metallic base or a polymeric base.

5. The system of claim 4 wherein the metallic base is selected from the group consisting of stainless steel, nitinol, tantalum, MP35N alloy, platinum, titanium, a suitable biocompatible alloy, a suitable biocompatible material, and a combination thereof.

6. The system of claim 1 wherein the therapeutic agent (126) is dispersed within the blended matrix of the polysulfone and the styrenic block copolymer.

7. The system of claim 1 wherein the polysulfone has a molecular weight between 10,000 Daltons and 100,000 Daltons.

8. The system of claim 1 wherein the styrenic block copolymer has a molecular weight between 200 Daltons and 200,000 Daltons

9. The system of claim 1 wherein the polymeric coating (124) comprises between 0.0 percent and 50 percent of the therapeutic agent by weight.

10. The system of claim 1 wherein the polymeric coating (124) has a thickness between 0.5 microns and 20 microns.

11. The system of claim 1 wherein the polymeric coating (124) has a weight between 50 micrograms and 1500 micrograms.

12. The system of claim 1 wherein the therapeutic agent (126) is positioned between the polymeric coating (124) and the stent framework (122).

13. The system of claim 12 wherein the therapeutic agent (126) positioned between the polymeric coating (124) and the stent framework (122) has a thickness between 0.1 microns and 20 microns.

14. The system of claim 1 wherein the blended matrix of the polysulfone and the styrenic block copolymer provides a controlled elution rate for the therapeutic agent (126).

15. The system of claim 1 wherein the therapeutic agent (126) is selected from the group consisting of an antirestenotic drug, an antisense agent, an antineoplastic agent, an antiproliferative agent, an antithrombogenic agent, an anticoagulant, an antiplatelet agent, an antibiotic, an anti-inflammatory agent, a steroid, a gene therapy agent, a therapeutic substance, an organic drug, a pharmaceutical compound, a recombinant DNA product, a recombinant RNA product, a collagen, a collagenic derivative, a protein, a protein analog, a saccharide, a saccharide derivative, a bioactive agent, a pharmaceutical drug, and a combination thereof.

16. The system of claim 1 wherein the polymeric coating (124) comprises a plurality of therapeutic agents (126), each therapeutic agent having a predetermined elution rate, the blended matrix of the polysulfone and the styrenic block copolymer eluting the therapeutic agents at the predetermined elution rates.

17. The system of claim 16 wherein a first therapeutic agent (126) is concentrated adjacent to the stent framework (122), and a second therapeutic agent (126) is concentrated adjacent to the outer surface of the polymeric coating (124).

18. The system of claim 17 wherein the first therapeutic agent (126) comprises an antirestenotic drug and the second therapeutic agent (126) comprises an anti-inflammatory drug.

19. The system of claim 1 further comprising:
a primer coating (228) disposed on the stent framework (122) between the stent framework and the polymeric coating (124).

20. The system of claim 19 wherein the primer coating (228) is selected from the group consisting of parylene, polyurethane, phenoxy, epoxy, polyimide, polysulfone, pellathane, and a suitable polymeric primer material.

21. A method of manufacturing a drug-polymer coated stent, comprising:
forming a polymeric solution including a styrenic block copolymer and a styrenic block copolymer solvent;
adding a polysulfone to the polymeric solution to form a blended matrix of the polysulfone and the styrenic block copolymer;
applying the polymeric solution onto a stent framework (122); and
drying the polymeric solution.

22. The method of claim 21 wherein the styrenic block copolymer solvent is selected from the group consisting of chloroform, methyl ethyl ketone, tetrahydrofuran, methyl chloride, toluene, ethyl acetate, dioxane, and a suitable organic solvent.

23. The method of claim 21 wherein the polymeric solution is applied using an application technique selected from the group consisting of dipping, spraying, painting, and brushing.

24. The method of claim 21 wherein the polymeric solution is dried in a vacuum environment.

25. The method of claim 21 wherein the polymeric solution is dried at a temperature between 25 degrees centigrade and 45 degrees centigrade.

26. The method of claim 21 further comprising:
mixing at least one therapeutic agent (126) with the polymeric solution prior to applying the polymeric solution onto the stent framework.

27. The method of claim 21 further comprising:
applying a therapeutic agent (126) to the stent framework prior to applying the polymeric solution onto the stent framework.

28. The method of claim 21 further comprising:
applying a primer coating (228) onto the stent framework prior to applying the polymeric solution onto the stent framework.

29. A drug-polymer coated stent, comprising:
a stent framework (122); and
a polymeric coating (124) disposed on the stent framework, wherein the polymeric coating comprises a blended matrix of a polysulfone and a styrenic block copolymer; and
a therapeutic agent (126) contacting the polymeric coating.

30. The stent of claim 29 wherein the stent framework (122) comprises one of a metallic base or a polymeric base.

31. The stent of claim 29 wherein the blended matrix comprises a chain length of the polysulfone and a chain length of the styrenic block copolymer based on a predetermined elution rate of the therapeutic agent.

32. The stent of claim 29 wherein the blended matrix comprises a first fraction of the polysulfone and a second fraction of the styrenic block copolymer based on a predetermined elution rate of the therapeutic agent.

33. The stent of claim 29 wherein the therapeutic agent (126) is selected from the group consisting of an antirestenotic agent, an antisense agent, an antineoplastic agent, an antiproliferative agent, an antithrombogenic agent, an anticoagulant, an antiplatelet agent, an antibiotic, an anti-inflammatory agent, a steroid, a gene therapy agent, a therapeutic substance, an organic drug, a pharmaceutical compound, a recombinant DNA product, a recombinant RNA product, a collagen, a collagenic derivative, a protein, a protein analog, a saccharide, and a saccharide derivative.

34. The stent of claim 29 wherein the therapeutic agent (126) is dispersed within the blended matrix of the polysulfone and the styrenic block copolymer.

35. The stent of claim 29 wherein the therapeutic agent (126) is positioned between the polymeric coating (124) and the stent framework (122).

36. The stent of claim 29 further comprising:
a primer coating (228) disposed on the stent framework (122) between the stent framework and the polymeric coating (124).

37. The stent of claim 29 wherein the primer coating (228) is selected from the group consisting of parylene, polyurethane, phenoxy, epoxy, polyimide, polysulfone, pellathane, and a suitable polymeric primer material.
